# EUROPEAN PATENT SPECIFICATION

(11) **EP 1 471 045 B1**
(45) Date of publication and mention of the grant of the patent: **08.01.2014**
(21) Application number: 02710363.9
(22) Date of filing: 29.01.2002
(51) Int. Cl.: C07C 37/20, C07C 39/17, C07C 41/30, C07C 43/23, C07B 61/00, C08G 59/04, C08G 63/199, C08G 64/06

(54) **PROCESS FOR PRODUCING FLUORENE DERIVATIVE**
VERFAHREN ZUR HERSTELLUNG VON FLUORENDERIVATEN
PROCEDE PERMETTANT DE PRODUIRE UN DERIVE DE FLUORENE

(43) Date of publication of application: 27.10.2004
(73) Proprietor: Osaka Gas Company Limited, Osaka-shi Osaka 541-0046 (JP)
(72) Inventor: MURASE, Hiroaki, c/o Osaka Gas Company Limited, Osaka-shi, Osaka 541-0046 (JP); YAMADA, Mitsuaki, c/o Osaka Gas Company Limited, Osaka-shi, Osaka 541-0046 (JP); SUDA, Yasuhiro, c/o Osaka Gas Company Limited, Osaka-shi, Osaka 541-0046 (JP); OGATA, Kazuyuki, c/o Osaka Gas Company Limited, Osaka-shi, Osaka 541-0046 (JP)
(74) Representative: Diehl & Partner GbR
(86) International application number: PCT/JP2002/000634
(87) International publication number: WO 2003/064358

(56) References cited:
- EP-A- 0 728 723
- JP-A- 2000 026 349
- JP-A- 2001 206 862
- JP-A- 2001 206 863
- JP-A- 2002 047 227

## Description

### TECHNICAL FIELD

The present invention relates to a method for producing a fluorene derivative useful as a raw material for optical lenses, films, optical fibers, optical disks, heat-resisting resins, engineering plastics, and others.

### BACKGROUND ART

Recently, in polymers (e.g., a polycarbonate-series resin, an epoxy resin, and a polyester-series resin) made from a bisphenol compound as a raw material, materials having improved heat resistance, transparency and high refractive index compared with the conventional one have been strongly required. A 9,9-bis(4-hydroxyphenyl)fluorene, which is one of fluorene derivatives, is a promising material for producing a polymer being excellent in heat resistance and having high transparency and high refractive index, and is expected as a raw material for an optical lens such as an automotive headlamp lens, a compact disk (CD), a CD-ROM pickup lens, a Fresnel lens, a fθ lens for laser printer, a camera lens, and a projection lens for rear projection television; a film such as a retardation film, and a diffusion film; a plastic optical fiber; and an optical disk substrate.

As a synthetic method for 9,9-bis(4-hydroxyphenyl)fluorene, there has been known a method which comprises subjecting fluorenone, as a starting material, obtained by air oxidation of fluorene to a condensation reaction with phenol using a hydrogen chloride gas and mercaptopropionic acid as catalysts [J. Appl. Polym. Sci., 27(9), 3289, 1982, Japanese Patent Application Laid-Open No. 145087/1994 (JP-6-145087), Japanese Patent Application Laid-Open No. 217713/1996 (JP-8-217713)].

However, since the reaction is dehydration reaction, it is necessary to provide (or set up) a special hydrogen chloride gas-generating apparatus and a special hydrogen chloride gas-removing apparatus when the reaction is industrially conducted using a gaseous hydrogen chloride, which has handling difficulty, as an acid catalyst. Moreover, the handling of the hydrogen chloride gas is under the control of various laws such as Fire Defense Law, High Pressure Gas Control Law, Poisonous and Deleterious Substances Control Law, and Clean Air Act Law. It is therefore necessary to pay sufficient attention to safety measures and environmental protection for installation of equipment, and handling and storage of the hydrogen chloride gas.

Incidentally, in the case using a concentrated sulfuric acid as an acid catalyst, the reaction is progressed by the dehydration property of the concentrated sulfuric acid despite of the presence of water. However, since a sulfuric acid-containing waste fluid is discharged in large quantity, the treatment of the waste fluid requires a great deal of labor.

On the other hand, a fluorene derivative obtainable by a production method using a hydrogen chloride gas or a concentrated sulfuric acid as a catalyst usually includes impurities such as a sulfonated compound, and turns out yellow. Accordingly, in order to use the fluorene derivative obtained by the method, as a raw material for the above-described polycarbonate-series resin or polyester-series resin in which high transparency is required, it is necessary to purify the fluorene derivative highly and strictly. Therefore, various purification methods have been examined [e.g., Japanese Patent Application Laid-Open No. 321836/1994 (JP-6-321836)]. However, such a purification is a factor in increased producing costs because of using a large amount of a solvent and making the production process longer.

EP-A-728723 describes a method for preparing 9,9-bis-(4-hydroxyphenyl)-fluorene by reacting fluorenone with phenol in the presence of a mecaptocarboxylic acid in form of β-mercaptopropionic acid or mercaptoacetic acid in a strongly acidic medium. As the acidic medium, there is used hydrochloric acid. However, the amounts of the reactants as used in the known method lead to purities, yields, colours and transparency properties, which are still unsatisfactory.

It is therefore an object of the present invention to provide a method for producing a fluorene derivative safely and simply at high yield without using a hydrogen chloride gas having handling difficulty.

It is another object of the present invention to provide a method for producing a highly transparent and highly purified fluorene derivative safely and simply without complicated purification.

### DISCLOSURE OF THE INVENTION

The inventors of the present invention made intensive studies to achieve the above objects in view of few problems of the conventional art mentioned above, and finally found that a fluorene derivative being scarcely colored and excellent in transparency can be simply or conveniently obtained by using a hydrochloric acid aqueous solution instead of a hydrogen chloride gas, and carrying out a reaction in coexistence with a thiol compound.

That is, in the present invention, fluorenone and a phenolic compound represented by the formula (I) are subjected to a condensation reaction in coexistence with a thiol compound and a hydrochloric acid aqueous solution to produce a fluorene derivative represented by the formula (II): 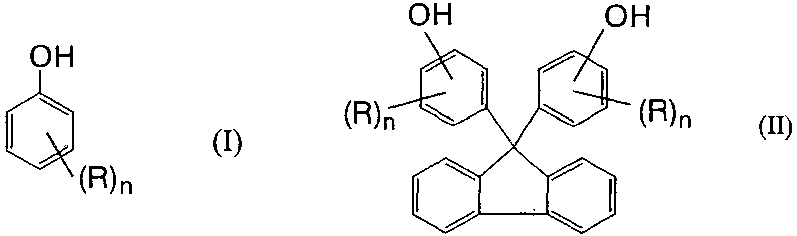 wherein R represents an alkyl group, an alkoxy group, an aryl group or a cycloalkyl group, and n denotes an integer of 0 to 4.

The phenolic compound includes a 2-C₁₋₄alkylphenol, and others. In the case using a mercaptocarboxylic acid (particularly β-mercaptopropionic acid) as the thiol compound, a fluorene derivative being scarcely colored and excellent in transparency is obtained. Moreover, the amount of the thiol compound is preferably larger than the amount of so-called catalyst. The proportion (weight ratio) of fluorenone relative to the thiol compound is 1/0.05 to 1/0.3. The proportion (weight ratio) of the thiol compound relative to hydrochloric acid (hydrogen chloride, HCl) contained in the hydrochloric acid aqueous solution [the thiol compound/hydrogen chloride] is 1/0.1 to 1/3, and preferably 1/0.3 to 1/2. The fluorene derivative includes a 9,9-bis(C₁₋₄alkylhydroxyphenyl)fluorene, in particular a 9,9-bis(4-hydroxy-3-C₁₋₄-alkylphenyl)fluorene, and the like.

Incidentally, in the method of present invention, an object compound may be crystallized by adding an extractant to a reaction mixture to distribute the object compound to an organic layer, and adding a crystallization solvent to the organic layer.

The method of the present invention ensures remarkable decrease in a yellowness or colored degree of a fluorene derivative by one (or a single) crystallizing operation, compared with conventional production methods.

### BEST MODE FOR CARRYING OUT THE INVENTION

The production method of a fluorene derivative of the present invention is to subject fluorenone and a phenolic compound to a condensation reaction in coexistence with a thiol compound and a hydrochloric acid aqueous solution.

### [Fluorene derivative]

In the fluorene derivative represented by the formula (II), R represents an alkyl group, a cycloalkyl group, an alkoxy group or an aryl group, and n denoted an integer of 0 to 4 (preferably 0 to 3, more preferably 0 to 2, and in particular 0 or 1). Incidentally, the kind of the substituent R may vary with n expressing the number of the substituent.

Examples of the alkyl group include a C₁₋₄alkyl group such as methyl group, ethyl group, propyl group, isopropyl group, n-butyl group, iso-butyl group, s-butyl group, and t-butyl group.

The cycloalkyl group includes a C₄₋₈cycloalkyl group such as cyclopentyl group and cyclohexyl group (preferably a C₅₋₆cycloalkyl group).

As the alkoxy group, there may be mentioned a C₁₋₄alkoxy group such as methoxy group, ethoxy group, propoxy group, n-butoxy group, iso-butoxy group, and tert-butoxy group.

The aryl group includes a C₁₋₄alkylphenyl group such as phenyl group, 2-methylphenyl group, 3-methylphenyl group, 4-methylphenyl group, 2,6-dimethylphenyl group, and 3,5-dimethylphenyl group, naphthyl group, and others.

The group R is preferably an alkyl group (e.g., a C₁₋₄alkyl group, particularly methyl group), a cycloalkyl group (e.g., cyclohexyl group), an aryl group (e.g., phenyl group).

The positions of hydroxyl group and the substituent R on the benzene ring are not particularly limited to a specific one. For example, hydroxyl group may be substituted on any of 2-position, 3-position and 4-position of the benzene ring, and is preferably substituted on 4-position of the benzene ring. The position(s) substituted by the substituent R varies with the number n, and for example, includes 2-position, 3-position, 4-position, 2,3-positions, 2,4-positions, 2,6-positions, 3,4-positions, and 3,5-positions of the benzene ring. The substituent R is preferably substituted on 2-position, 3-position and 3,5-position of the benzene ring, and more preferably substituted on 3-position of the benzene ring.

Specific examples of the fluorene derivative include 9,9-bis(4-hydroxyphenyl)fluorene; a 9,9-bis(alkylhydroxyphenyl)fluorene such as 9,9-bis(4-hydroxy-2-methylphenyl)fluorene, 9,9-bis(4-hydroxy-3-methylphenyl)fluorene, 9,9-bis(4-hydroxy-3-ethylphenyl)fluorene, 9,9-bis(3-hydroxy-6-methylphenyl)fluorene, 9,9-bis(2-hydroxy-4-methylphenyl)fluorene, and 9,9-bis(4-hydroxy-3-t-butylphenyl)fluorene; a 9,9-bis(dialkylhydroxyphenyl)fluorene such as 9,9-bis(4-hydroxy-3,5-dimethylphenyl)fluorene, 9,9-bis(4-hydroxy-2,6-dimethylphenyl)fluorene, and 9,9-bis(4-hydroxy-3,5-di-tert-butylphenyl)fluorene; a 9,9-bis(cycloalkylhydroxyphenyl)fluorene such as 9,9-bis(4-hydroxy-3-cyclohexylphenyl)fluorene; a 9,9-bis(arylhydroxyphenyl)fluorene such as 9,9-bis(4-hydroxy-3-phenylphenyl)fluorene; and others.

Among these fluorene derivatives, a 9,9-bis(hydroxyphenyl)fluorene, a 9,9-bis(C₁₋₄ alkylhydroxyphenyl)fluorene, and a 9,9-bis(hydroxyarylphenyl)fluorene, in particular a 9,9-bis(4-hydroxy-3-C₁₋₄alkylphenyl)fluorene [e.g., 9,9-bis(4-hydroxy-3-methylphenyl)fluorene], are preferred.

### [Fluorenone]

The purity of fluorenone is not particularly limited to a specific one, and is usually not less than 95% by weight and preferably not less than 99% by weight.

### [Phenolic compound]

The phenolic compound is represented by the above-mentioned formula (I). The group R and the number n in the formula (I) have the same meanings as defined in the above formula (II).

Specific examples of the phenolic compound include phenol, an alkylphenol (a cresol such as o-cresol, m-cresol, and p-cresol), a dialkylphenol (e.g., 2,3-dimethylphenol, 2,5-dimethylphenol, 2,6-dimethylphenol, and 2,6-ditert-butylphenol), a trialkylphenol, an alkoxyphenol (e.g., an anisole such as o-methoxyphenol), an arylphenol (e.g., a phenylphenol such as o- or m-phenylphenol), a cycloalkylphenol (e.g., 2-cyclohexylphenol), and others. The phenolic compounds may be used singly or in combination. Among these phenolic compounds, a C₁₋₄alkylphenol, for example, a 2-C₁₋₄alkylphenol (e.g., o-cresol) is preferred.

The purity of the phenolic compound is not particularly limited to a specific one, and is usually not less than 95% by weight and preferably not less than 99% by weight.

From the viewpoint of high-yield production of the fluorene derivative and inhibition of side reactions, the phenolic compound is usually excessively employed relative to fluorenone. For example, the proportion (molar ratio) of fluorenone relative to the phenolic compound [fluorenone/the phenolic compound] is about 1/2 to 1/30, preferably about 1/3 to 1/20, and more preferably about 1/4 to 1/10. Incidentally, the phenolic compound may be employed in excess to use as a reaction solvent.

### [Hydrochloric acid aqueous solution]

The concentration of the hydrochloric acid aqueous solution (an aqueous solution of hydrochloric acid) as a catalyst is usually about 5 to 37% by weight (e.g., about 5 to 36% by weight), preferably about 10 to 37% by weight (e.g. , about 25 to 37% by weight), and particularly about 30 to 37% by weight (e.g. , about 35 to 36% by weight). The proportion (weight ratio) of fluorenone relative to the hydrochloric acid aqueous solution [fluorenone/the hydrochloric acid] is usually, on hydrochloric acid (hydrogen chloride, HCl) basis, about 1/0.01 to 1/1, preferably about 1/0.05 to 1/0.5, and more preferably about 1/0.1 to 1/0.3. Incidentally, since the reaction of fluorenone with the phenolic compound is a dehydration reaction, generally the catalytic activity cannot be effectively expressed by using the hydrochloric acid aqueous solution. However, in the case using a thiol compound in combination, the reaction effectively proceeds even using the hydrochloric acid aqueous solution.

### [Thiol compound]

As the thiol compound as a promoter, a conventional thiol compound may be used. The thiol compound includes, for example, a mercaptocarboxylic acid (e.g., thioacetic acid, β-mercaptopropionic acid, α-mercaptopropionic acid, thioglycolic acid, thiooxalic acid, mercaptosuccinic acid, and mercaptobenzoic acid), an alkyl mercaptan (e.g., a C₁₋₄alkyl mercaptan such as methyl mercaptan, ethyl mercaptan, propyl mercaptan, isopropyl mercaptan, and n-butyl mercaptan), an aralkyl mercaptan (e.g., benzyl mercaptan), or a salt thereof, and others. Examples of the salt include an alkali metal salt (e.g., a sodium salt). The thiol compounds may be used singly or in combination.

Among these thiol compounds, a mercaptocarboxylic acid (e.g., β-mercaptopropionic acid) is preferred.

The proportion (weight ratio) of fluorenone relative to the thiol compound is 1/0.05 to 1/0.3, and more preferably about 1/0.08 to 1/0.15. According to the production method of the present invention, since the thiol compound employed in combination with the hydrochloric acid aqueous solution is used in a larger amount compared to the amount of the catalyst usually employed, a fluorene derivative of high purity can be obtained at a high yield without using a hydrogen chloride gas which has handling difficulty.

The proportion (weight ratio) of the thiol compound relative to the hydrochloric acid aqueous solution, when the hydrochloric acid aqueous solution is converted into hydrochloric acid (hydrogen chloride, HCl), [the thiol compound/hydrogen chloride] is 1/0.1 to 1/3, preferably about 1/0.3 to 1/2, and more preferably about 1/0.5 to 1/1.5. According to the production method of the present invention, use of the thiol compound and hydrochloric acid at the above proportion ensures a simple (or convenient) production of a fluorene derivative excellent in transparency.

### [Production method of fluorene derivative]

The production process of the present invention may be conducted by charging fluorenone, a phenolic compound (I), a thiol compound, and the hydrochloric acid aqueous solution in a reactor, and stirring the mixture under an atmosphere of an inert gas. As the inert gas, for example, there may be utilized a nitrogen gas, an argon gas, and a helium gas.

The reaction temperature varies with the kind of a phenolic compound or a thiol compound to be used, and is usually about 10 to 100°C (e.g., about 10 to 80°C) and preferably about 20 to 50°C. When the reaction temperature is too low, the reaction rate becomes slow. When the reaction temperature is too high, a side reaction occurs and results in the yield deterioration.

The reaction may be carried out in the presence of a solvent such as toluene and xylene, and may be usually carried out in the absence of a solvent. Moreover, use of an excessive amount of the phenolic compound as a solvent can make the reaction more smoothly.

The progress of the reaction may be followed up by an analytical means such as a liquid chromatography, and the point that an amount of unreacted fluorenone becomes not more than 0.5% by weight in the reaction mixture may be determined as an end point. The reaction mixture after completion of the reaction usually contains unreacted fluorenone, an unreacted phenolic compound, the catalyst, by-product(s), and others in addition to a fluorene derivative as a reaction product.

After completion of the reaction, a highly purified fluorene derivative is obtained from the reaction mixture by a conventional manner (e.g., a means such as concentration, extraction, crystallization, filtration and chromatography, or a separation and purification means by combination thereof). In particular, by at least a crystallizing operation, especially by combination of a distributing operation and a crystallizing operation, a color-free fluorene derivative with a high purity can be obtained simply and easily. The distributing operation may for example be carried out by adding an extractant (an organic solvent alone, or a mixed solvent of an organic solvent and water) to the reaction mixture to transfer or extract the object compound into an organic layer. The crystallizing operation may be carried out by optionally condensing the organic layer, then adding a crystallization solvent to the organic layer, and optionally cooling the resultant mixture.

After removing the remaining hydrochloric acid and the thiol compound from the reaction mixture, a crystallization solvent is usually added to the residue for mixing and dissolving. More specifically, a purified fluorene derivative may be obtained by subjecting fluorenone and an excessive amount of a phenolic compound to a condensation reaction in coexistence with β-mercaptopropionic acid and hydrochloric acid, adding an extractant to the reaction mixture for distributing a fluorene derivative to an organic layer, condensing the organic layer, and adding a crystallization solvent to the residue to crystallize an object compound. Moreover, a fluorene derivative may be crystallized by neutralizing the reaction mixture with an alkaline aqueous solution to remove the aqueous layer, condensing the organic layer, and adding a crystallization solvent to the residue.

The neutralizing treatment may be conducted by adding at least an alkaline aqueous solution to the reaction mixture. As the alkali, there may be used an alkali metal hydroxide, an inorganic base (such as a carbonate) and/or an organic base. The neutralizing treatment may be carried out by adding both an extractant and an alkaline aqueous solution to the reaction mixture. Examples of the extractant include an organic solvent to which a fluorene derivative is soluble (e.g., a aliphatic hydrocarbon such as hexane, an aromatic hydrocarbon such as toluene and xylene, an alicyclic hydrocarbon such as cyclohexane, and a halogenated hydrocarbon), or if necessary, a mixed solvent of the organic solvent and a poor solvent to a fluorene derivative (e.g., water).

After the neutralizing treatment, the extractant layer (organic layer) is optionally washed with water, the water layer is removed, and then a crystallization solvent may be appropriately added to the organic layer to precipitate (or separate) a crystal. The fluorene derivative is usually crystallized by removing the organic solvent by distillation or other means to condense the organic layer, and adding a crystallization solvent to the residue. The crystallization solvent comprises a hydrocarbon and a polar solvent. It is estimated that the polar solvent produces a clathrate crystal with the fluorene derivative.

Examples of the hydrocarbon include an aliphatic hydrocarbon such as pentane, hexane, and octane; an alicyclic hydrocarbon such as cyclohexane, and methylcyclohexane; an aromatic hydrocarbon such as benzene, toluene, xylene, and ethylbenzene; a halogenated hydrocarbon such as dichloromethane, dichloroethane, trichloroethylene, and dichlorobenzene; and others. These hydrocarbons may be used singly or in combination. The preferred hydrocarbon includes a solvent to which a fluorene derivative is soluble, in particular, an aromatic hydrocarbon (e.g., toluene).

The polar solvent includes, for example, water, an alcohol (e.g., a C₁₋₄alcohol such as methanol, ethanol, propanol, isopropanol and butanol, in particular a C₁₋₃ alkylalcohol), a ketone (e.g., a diC₁₋₄alkyl ketone such as acetone, methyl ethyl ketone, methyl isobutyl ketone, diethyl ketone, ethyl propyl ketone, di-n-propyl ketone and diisopropyl ketone, in particular a C₃₋₇alkyl ketone), a nitrile (e.g., acetonitrile); and others. These polar solvents may be used singly or in combination. In particular, a crystallization solvent comprising the aromatic hydrocarbon (particularly toluene) and the ketone (particularly acetone) is effective in removing a causative substance of coloring (an impurity, a coloring component).

The proportion of the polar solvent relative to the hydrocarbon is, for example, about 0.5 to 10 parts by weight (e.g., about 1 to 10 parts by weight), preferably about 2 to 8 parts by weight (e.g., about 2 to 6 parts by weight), and particularly about 3 to 5 parts by weight, relative to 1 part by weight of the hydrocarbon. The amount of the crystallization solvent is usually about 1 to 10 parts by weight, preferably about 1 to 5 parts by weight (e.g., about 2 to 5 parts by weight), relative to 1 part by weight of the residue (or solid matter).

The crystallizing operation may be carried out by a conventional method, for example, by dissolving the residue in a crystallization solvent and cooling the mixture. A highly purified and highly transparent fluorene derivative is obtained by collecting (or recovering) a precipitated crystal by filtration or other means, optionally washing the crystal, and drying the crystal. Incidentally, the crystallizing operation may be repeatedly carried out. In the present invention, a fluorene derivative being scarcely colored and having high transparency can be obtained by one (or a single) crystallizing operation.

The production process of the present invention achieves a highly purified fluorene derivative practicable as a raw material for a polymer in which high transparency is required (e.g., a polycarbonate-series resin, a polyester-series resin, and an epoxy resin) . The b value in the Hunter color system of the obtained fluorene derivative is, for example, not more than 3, preferably not more than 2, and more preferably not more than 1.5, where the b value is determined from a transmittance measured by a visible and ultraviolet absorption apparatus (wavelength: 380 to 780 nm).

### INDUSTRIAL APPLICABILITY

A hydrochloric acid aqueous solution has not efficiently expressed the activity as an acid catalyst because water in the solution induces inhibition of the reaction, and thus a gaseous hydrogen chloride having handling difficulty has been used. According to the present invention, however, since such a hydrochloric acid aqueous solution can be used instead of the gaseous hydrogen chloride, as an acid catalyst, a fluorene derivative can be obtained safely and simply at high yield. Moreover, thus obtained fluorene derivative can be obtained by only one crystallization operation with high purity and high transparency, and can be used as a raw material for polymer, resulting in decreasing the purification cost.

### EXAMPLES

The following examples and comparative examples are intended to describe this invention in further detail and the examples should by no means be interpreted as defining the scope of the invention.

In the examples, the purity was expressed in percentage of the area by an analysis of a high performance liquid chromatography (manufactured by Waters Corporation) with a reversed layer column. Moreover, the b value was determined (or calculated) from a transmittance measured at a wavelength of 380 to 780 nm by a visible and ultraviolet absorption apparatus (manufactured by Hitachi, Ltd.). Further, the yield of a fluorene derivative was calculated based on a proportion (molar ratio) of the fluorene derivative relative to fluorenone.

### Example 1

In a 2 L glass vessel equipped with a stirrer, a cooler and a thermometer were charged fluorenone (75g) having a purity of 99% by weight, o-cresol (270g), β-mercaptopropionic acid (8.5g), and 36% by weight of hydrochloric acid aqueous solution (27g). The mixture was subjected to a reaction with stirring under an atmosphere of an inert gas at 25°C for 6 hours, followed by at 35°C for 11 hours. The analysis of the reaction product by HPLC showed that the remaining amount of fluorenone was not more than 0.1% by weight.

After toluene (300g) and water (80g) were added to the resultant reaction solution, an aqueous solution containing sodium hydroxide (32% by weight) was added to the mixture for neutralization to approximately pH 7, then the resulting water layer was removed. The organic layer was heated to 80°C, and washed with water (80g) three times.

After collecting toluene (300g) by distillation under a reduced pressure, a mixture (500 ml) containing toluene and acetone at a mixing ratio (weight ratio) [toluene/acetone] of 1/4 was added to the organic layer, and stirred at 70°C for one hour. Then, the resultant mixture was cooled to 10°C for crystallization to give an object product, 9,9-bis(4-hydroxy-3-methylphenyl)fluorene (140g, yield 89%).

The purity of thus obtained fluorene derivative was 99.6% by weight. Moreover, the b value was 1.3 (colorless and transparency). The fluorene derivative can be therefore used as a raw material of polymer without further crystallization operation.

### Example 2

A reaction was carried out in the same manner as in Example 1 except for using phenol (225g) instead of o-cresol (270g). As a result, an object product, 9,9-bis(4-hydroxyphenyl)fluorene, was obtained (127g, yield 87%).

The purity of thus obtained fluorene derivative was 99.3% by weight. Moreover, the b value was 1.7 (colorless and transparency). The fluorene derivative can be therefore used as a raw material of polymer without further crystallization operation.

### Example 3

A reaction was conducted in the same manner as in Example 1 except that o-phenylphenol (425g) was used instead of o-cresol (270g). As a result, an object product, 9,9-bis(4-hydroxy-3-phenylphenyl)fluorene, was obtained (185g, yield 90%).

The purity of thus obtained fluorene derivative was 99.0% by weight. Moreover, the b value was 1.8 (colorless and transparency), and the fluorene derivative can be therefore used as a raw material of polymer without further crystallization operation.

### Comparative Example 1

In a 2 L glass vessel equipped with a stirrer, a cooler, a thermometer and a tube for supplying hydrogen chloride gas were charged fluorenone (75g) having a purity of 99% by weight, o-cresol (160g), and β-mercaptopropionic acid (2g), and fluorenone was completely dissolved in the mixture with stirring and heating at 50°C under an atmosphere of an inert gas. A hydrogen chloride gas was passed through the mixture at a feed rate of 200 ml/minute, and initiated the reaction. The reaction was continued for 4 hours with maintaining the reaction temperature of 50°C. After complication of the reaction, a nitrogen gas was passed through the reaction mixture at a feed rate of 5 L/minute for 30 minutes to drive out the remaining hydrogen chloride gas in the vessel.

After toluene (300g) and water (80g) were added to thus obtained reaction solution, an aqueous solution containing sodium hydroxide of 32% by weight was added to the mixture for neutralization to approximately pH 7, then the water layer was removed. The organic layer was heated to 80°C, and washed with water (80g) three times.

After collecting toluene (300g) by distillation under a reduced pressure, a mixture (500 ml) containing toluene and acetone at a mixing ratio [toluene/acetone] of 1/4 (weight ratio) was added to the organic layer, and stirred at 70°C for one hour. Thereafter, the resultant mixture was cooled to 10°C for crystallization to give an object product, 9,9-bis(4-hydroxy-3-methylphenyl)fluorene (120g, yield 76%).

The purity of the resulting fluorene derivative was 95.2% by weight. Moreover, the b value was 12.5 (light yellow), and it was necessary to operate crystallization further 3 times under the same conditions in order to obtain the fluorene derivative having the b value of not more than 3 which was required to use as a raw material for a polymer.

### Comparative Example 2

A reaction was conducted in the same manner as in Comparative Example 1 except that phenol (133g) was used instead of o-cresol (160g). As a result, an object product, 9,9-bis(4-hydroxyphenyl)fluorene, was obtained (101g, yield 69%).

The purity of thus obtained fluorene derivative was 93.9% by weight. Moreover, the b value was 18.5 (light yellow), and it was necessary to operate crystallization further 3 times under the same conditions in order to obtain the fluorene derivative having the b value of not more than 3 which was required to use as a raw material for a polymer.

### Comparative Example 3

A reaction was conducted in the same manner as in Comparative Example 1 except that o-phenylphenol (252g) was used instead of o-cresol (160g). As a result, an object product, 9,9-bis(4-hydroxy-3-phenylphenyl)fluorene, was obtained (113g, yield z).

The purity of the resulting fluorene derivative was 96.1% by weight. Moreover, the b value was 19.3 (light yellow), and it was necessary to operate crystallization further 3 times under the same conditions in order to obtain the fluorene derivative having the b value of not more than 3 which was required to use as a raw material for a polymer.

## Claims

1. Method for producing a fluorene derivative, which comprises subjecting fluorenone and a phenolic compound represented by the formula I 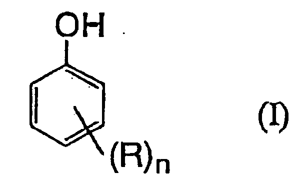 wherein R represents an alkyl group, an alkoxy group, an aryl group or a cycloalkyl group, and n denotes an integer of 0 to 4,
to a condensation reaction in coexistence with a mercaptocarboxylic acid and hydrochloric acid to obtain a fluorene derivative represented by the formula II 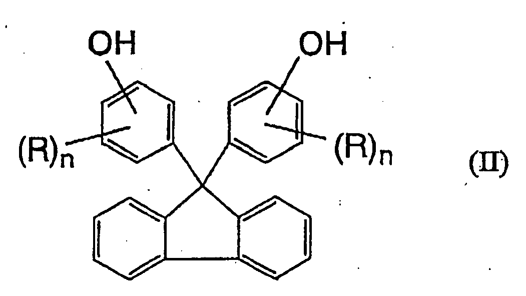 wherein R and n have the same meanings as defined above, **characterized in that**
the weight ratio of the mercaptocarboxylic acid relative to hydrogen chloride contained in the hydrochloric acid is 1/0.1 to 1/3, the weight ratio of fluorenone relative to the mercaptocarboxylic acid is 1/0.05 to 1/0.3, and an extractant is added to the resulting condensation reaction mixture to distribute the object compound into the organic layer as well as a crystallization solvent is added to the organic layer to crystallize the fluorene derivative.

2. Method according to claim 1, wherein the phenolic compound represented by formula I comprises phenol or a C₁₋₄-alkylphenol.

3. Method according to claim 1, wherein the phenolic compound represented by formula I comprises a 2-C₁₋₄-alkylphenol or a 3-C₁₋₄-alkylphenol.

4. Method according to claim 1, wherein the weight ratio of the mercaptocarboxylic acid relative to hydrogen chloride contained in the hydrochloric acid is 1/0.3 to 1/2.

5. Method according to claim 1, wherein the fluorene derivative represented by formula II comprises a 9. 9-bis(C₁₋₄-alkylhydroxyphenyl) fluorene.

6. Method for producing a 9,9-bis(4-hydroxy-3-C₁₋₄-alkyl-phenyl)fluorene, **characterized by** subjecting fluorenone and a 2-C₁₋₄-alkylphenol to a condensation reaction in coexistence with β-mercaptopropionic acid and hydrochloric acid, wherein the weight ratio of β-mercaptopropionic acid relative to hydrogen chloride contained in the hydrochloric acid is 1/0.1 to 1/3, the weight ratio of fluorenone relative to the mercaptocarboxylic acid is 1/0.05 to 1/0.3, and an extractant is added to the resulting condensation reaction mixture to distribute the object compound into the organic layer as well as a crystallization solvent is added to the organic layer to crystallize the fluorene derivative.

## Patentansprüche

1. Verfahren zum Herstellen eines Fluorenderivats, umfassend das Unterziehen von Fluorenon und einer Phenolverbindung, die durch die folgende Formel I dargestellt wird: 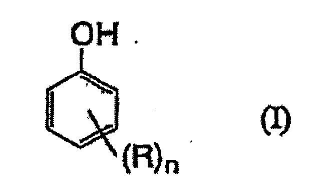 worin R für eine Alkylgruppe, eine Alkoxygruppe, eine Arylgruppe oder eine Cycloalkylgruppe steht und n eine ganze Zahl von 0 bis 4 bezeichnet,
einer Kondensationsreaktion in Koexistenz mit einer Mercaptocarbonsäure und Salzsäure, um ein Fluorenderivat zu erhalten, das durch die folgende Formel II dargestellt wird: 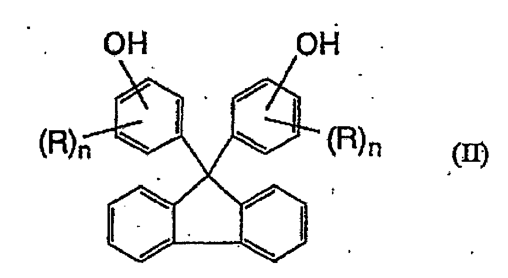 worin R und n die gleichen Bedeutungen wie oben definiert haben, **dadurch gekennzeichnet, dass**
das Gewichtsverhältnis der Mercaptocarbonsäure zu dem Chlorwasserstoff, der in der Salzsäure enthalten ist, 1/0,1 bis 1/3 beträgt, das Gewichtsverhältnis von Fluoren zu der Mercaptocarbonsäure 1/0,05 bis 1/0,3 beträgt und ein Extraktionsmittel zu der resultierenden Kondensationsreaktionsmischung gegeben wird, um die Ziel-Verbindung in der organischen Schicht zu verteilen, sowie ein Kristallisationslösungsmittel zu der organischen Schicht gegeben wird, um das Fluorenderivat zu kristallisieren.

2. Verfahren gemäß Anspruch 1, wobei die Phenolverbindung, die durch Formel I dargestellt ist, Phenol oder ein C₁₋₄-Alkylphenol umfasst.

3. Verfahren gemäß Anspruch 1, wobei die Phenolverbindung, die durch Formel I dargestellt wird, ein 2-C₁₋₄-Alkylphenol oder ein 3-C₁₋₄-Alkylphenol umfasst.

4. Verfahren gemäß Anspruch 1, wobei das Gewichtsverhältnis der Mercaptocarbonsäure zu dem Chlorwasserstoff, der in der Salzsäure enthalten ist, 1/0,3 bis 1/2 beträgt.

5. Verfahren gemäß Anspruch 1, wobei das Fluorenderivat, das durch die Formel II dargestellt wird, ein 9,9-Bis(C₁₋₄-alkylhydroxyphenyl)fluoren umfasst.

6. Verfahren zum Herstellen eines 9,9-Bis(4-hydroxy-3-C₁₋₄-alkyl-phenyl)fluorens, **gekennzeichnet durch** das Unterziehen von Fluoren und einem 2-C₁₋₄-Alkylphenol einer Kondensationsreaktion in Koexistenz mit β-Mercaptopropionsäure und Salzsäure, wobei das Gewichtsverhältnis von β-Mercaptopropionsäure zu Chlorwasserstoff, der in der Salzsäure enthalten ist, 1/0,1 bis 1/3 beträgt, wobei das Gewichtsverhältnis von Fluorenon zu der Mercaptocarbonsäure 1/0,05 bis 1/0,3 beträgt und ein Extraktionsmittel zu der resultierenden Kondensationsreaktionsmischung gegeben wird, um die Ziel-Verbindung in der organischen Schicht zu verteilen sowie ein Kristallisationslösungsmittel zu der organischen Schicht gegeben wird, um das Fluorenderivat zu kristallisieren.

## Revendications

1. Procédé pour produire un dérivé de fluorène, qui comprend la soumission de fluorénone et d'un composé phénolique représenté par la formule I 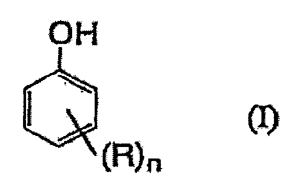 où R représente un groupe alkyle, un groupe alcoxy, un groupe aryle ou un groupe cycloalkyle, et n désigne un nombre entier de 0 à 4,
à une réaction de condensation en coexistence avec un acide mercaptocarboxyli.que et un acide chlorhydrique pour obtenir un dérivé de fluorène représenté par la formule II 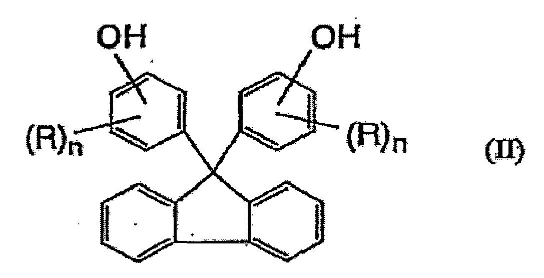 où R et n ont les mêmes significations que celles définies ci-dessus, **caractérisé en ce que**
le rapport en poids de l'acide mercaptocarboxylique sur le chlorure d'hydrogène contenu dans l'acide chlorhydrique est de 1/0,1 à 1/3, le rapport en poids du fluorénone sur l'acide mercaptocarboxylique est de 1/0,05 à 1/0,3, et un agent d'extraction est ajouté au mélange réactionnel de condensation résultant pour distribuer le composé en objet dans la couche organique alors qu'un solvant de cristallisation est ajouté à la couche organique pour cristalliser le dérivé de fluorène.

2. Procédé selon la revendication 1, dans lequel le composé phénolique représenté par la formule I comprend du phénol ou un alkylphénol en C₁-C₄.

3. Procédé selon la revendication 1, dans lequel le composé phénolique représenté par la formule I comprend un 2-alkylphénol en C₁-C₄ ou un 3-alkylphénol en C₁-C₄.

4. Procédé selon la revendication 1, dans lequel le rapport en poids de l'acide mercaptocarboxylique sur le chlorure d'hydrogène contenu dans l'acide chlorhydrique est de 1/0,3 à 1/2.

5. Procédé selon la revendication 1, dans lequel le dérivé de fluorène représenté par la formule II comprend un 9, 9-bis (alkylhydroxyphényl en C₁-C₄) fluorène.

6. Procédé pour produire un 9,9-bis(4-hydroxy-3-alkylphényl en C₁-C₄) fluorène, **caractérisé par** la soumission du fluorénone et d'un 2-alkylphénol en C₁-C₄ à une réaction de condensation en coexistence avec de l'acide β-mercaptopropionique et de l'acide chlorhydrique, dans lequel le rapport en poids de l'acide β-mercaptopropionique sur le chlorure d'hydrogène contenu dans l'acide chlorhydrique est de 1/0,1 à 1/3, le rapport en poids du fluorénone sur l'acide mercaptocarboxylique est de 1/0,05 à 1/0,3, et un agent d'extraction est ajouté au mélange réactionnel de condensation résultant pour distribuer le composé en objet dans la couche organique alors qu'un solvant de cristallisation est ajouté à la couche organique pour cristalliser le dérivé de fluorène.
